# EUROPEAN PATENT SPECIFICATION

(11) **EP 3 482 206 B1**
(45) Date of publication and mention of the grant of the patent: **01.04.2020**
(21) Application number: 17765490.2
(22) Date of filing: 05.07.2017
(51) Int. Cl.: G01N 33/574

(54) **METHOD FOR IN VITRO DIAGNOSIS OF THYROID CARCINOMA**
VERFAHREN ZUR IN-VITRO-DIAGNOSE VON SCHILDDRÜSENKARZINOM
PROCÉDÉ DE DIAGNOSTIC IN VITRO DU CARCINOME THYROÏDIEN

(30) Priority: 05.07.2016 IT 201600069927
(43) Date of publication of application: 15.05.2019
(73) Proprietor: Fondazione Università Niccolò Cusano Per la Ricerca Medico Scientifica, 00166 Roma (IT)
(72) Inventor: TROVATO, Maria Concetta, 00166 Roma (IT); SCIACCHITANO, Salvatore, 00166 Roma (IT)
(74) Representative: Gitto, Serena
(86) International application number: PCT/IT2017/000137
(87) International publication number: WO 2018/008048

(56) References cited:
- WO-A1-2008/085007
- WO-A1-2015/100257
- WO-A2-2011/143361
- KRZYSZTOF FUJAREWICZ ET AL: "A multi-gene approach to differentiate papillary thyroid carcinoma from benign lesions: gene selection using support vector machines with bootstrapping", ENDOCRINE - RELATED CANCER, BIOSCIENTIFICA LTD, GB, vol. 14, 1 January 2007 (2007-01-01), pages 809-826, XP007915082, ISSN: 1351-0088, DOI: 10.1677/ERC-06-0048
- ZHIWEI WANG ET AL: "Roles of F-box proteins in cancer", NATURE REVIEWS. CANCER, vol. 14, no. 4, 24 March 2014 (2014-03-24) , pages 233-247, XP055374507, GB ISSN: 1474-175X, DOI: 10.1038/nrc3700
- RANDLE SUZANNE J ET AL: "F-box protein interactions with the hallmark pathways in cancer", SEMINARS IN CANCER BIOLOGY, SAUNDERS SCIENTIFIC PUBLICATIONS, PHILADELPHIA, PA, US, vol. 36, 28 September 2015 (2015-09-28), pages 3-17, XP029422098, ISSN: 1044-579X, DOI: 10.1016/J.SEMCANCER.2015.09.013

## Description

The present invention concerns a method for *in vitro* diagnosis of thyroid carcinoma. Specifically, the present invention concerns a method for *in vitro* diagnosis of thyroid carcinoma by detecting the localization of FBXO34 protein.

Benign and malignant thyroid neoplasms appear through pathological features of thyroid nodules (TN)(1). Nodular lesions are quite common in the general population. A single or multiple thyroid nodules can be observed in approximately 10% of worldwide adult population. It has been reported that 10-18 million of individuals in the USA are affected. The occurrence of thyroid nodules increases with age and they are more frequent in women compared to men (4.3% versus 1.2%) (2). A malignancy can be detected in 7% of solitary thyroid nodule while its occurrence in multinodular glands is around 9% (3). The incidence of thyroid carcinomas (TC) has continuously increased over the last three decades and this trend is observed in every continent (4). In women, TC is the fifth most common cancer and in Italy it is the second most frequent cancer diagnosed in women under 45 years old (5).

A reliable, early and definite diagnosis of malignant nodules is the diagnostic goal of any clinician facing a thyroid nodule. However, the accuracy of clinical, radiological, scintigraphic and even cytological criteria may be unsatisfactory and many unnecessary surgeries are still performed to remove benign lesions. This is confirmed by recent epidemiological data reporting that 42-77% of TN removed are benign by histology examination and therefore, surgical excision could be evitable (6, 7). To date cyto-pathological examination represents the best available way to distinguish benign from malignant thyroid lesions in pre-operatory stage and then, the best analysis to adequately alert patients toward surgery (8). In fact, FNAB recognizes a malignant lesion in 96-98% of nodules classified as Thy5 (according to the British Thyroid Association) and in 68-70% of nodules classified as Thy4. In nodules classified as Thy3 the reported frequency of malignancy is 9.5-43%. The occurrence of misdiagnosed cancer in lesions classified as benign (Thy2) is 3-5.8% while in case the cytological sample is not adequate for a diagnosis (Thy1) the occurrence of malignancy is 4.5-8.5%. Therefore, the major limit of thyroid FNAB is represented by the category of Thy3, the so-called "indeterminate" samples, usually encountered in 15-30% of nodules aspirated (9-11). In the absence of valid indications, very often these indeterminate cases undergo unnecessary surgery (12).

In the light of the above it is therefore apparent the need to provide for new methods for diagnosis of thyroid carcinoma able to overcome the disadvantages of known diagnostic methods.

FBXO34 is a 78,711 Da molecular mass protein, including 711 amino acids and belonging to F-box protein family, which include a total of 39 members (13). The common genetic trait of this family is the F-box protein motif, consisting in approximately 40 amino acids. This motif was initially recognized in budding yeast Cdc4p and human Ciclyn F (14). F-box proteins are best known for their role as the substrate-recognition components of the SKP1/CUL1/F-box protein (SCF) class of E3 ubiquitin ligases (13, 15). Some F-box proteins are distributed both in the cytoplasm and in the nucleus. The identical localization of wild-type and mutant F-box proteins demonstrates that the presence of the F-box and the F-box-dependent binding to Skp1 does not determine the subcellular localization of these proteins (16). The tissue and cellular distribution as well as the specific role of FBXO34 in cell proliferation and in cancer development or progression are not known.

According to the present invention, it has been found that FBXO34 shows a double localization in malignant follicular and parafollicular thyroid cells, i.e. it appears both in nucleus and cytoplasm, whereas it shows a unique nuclear localization in normal and benign follicular thyroid cells. The results are based on three different lines of evidences, namely western blot analysis, immunoflorescent experiments and immunohistochemistry. The experiments were performed *"in vitro"* on human thyroid cancer cell lines and *"ex vivo"* in surgically excised human thyroid tissue samples.

The delocalization of FBOX34 protein in the cytoplasm, which has been observed in thyroid cancer, allows setting up a new analytic diagnostic method to distinguish directly benign from malignant lesions by analyzing cytological samples obtained by FNA. The subcellular immunohistochemical expressions of FBOX34 represent, therefore, a new diagnostic marker able to further ameliorate the recognition of thyroid cancer especially in those lesions that remain "indeterminate" after conventional cytological analysis.

It is therefore specific object of the present invention the use of FBXO34 protein as a marker of thyroid carcinoma, wherein said protein is localized in both cytoplasm and nucleus of thyroid cells in thyroid carcinoma. The localization of the protein can be detected *in vitro* by the use of a thyroid biological sample.

Thyroid carcinoma that can be detected is for example papillary, follicular, anaplastic or medullary thyroid carcinoma. The thyroid cells to be observed are therefore follicular and/or parafollicular cells. Whereas thyroid biological samples can be thyroid cells or tissues obtained for example by fine needle aspiration or by surgery.

The present invention concerns also a method for *in vitro* diagnosis of thyroid carcinoma, said method comprising or consisting of detecting the localization of FBXO34 protein in cytoplasm (pathological localization), and optionally in nucleus (physiological localization), of thyroid cells in a thyroid biological sample, wherein said protein is localized in both the cytoplasm (pathological localization) and the nucleus (physiological localization) of malignant thyroid cells.

Therefore, when only the localization in cytoplasm is observed, FBXO34 protein is detected in thyroid carcinoma, whereas it is not detected in normal or benign thyroid cells. When both the localizations in cytoplasm and in nucleus are observed, FBXO34 protein is detected both in the cytoplasm and in the nucleus of thyroid cells in thyroid carcinoma, whereas it is detected only in the nucleus of normal or benign thyroid cells.

According to the present invention, thyroid carcinoma can be chosen from the group consisting of papillary, follicular, anaplastic and medullary carcinoma. Therefore, thyroid cells to be analyzed are for example follicular and/or parafollicular cells. In fact, the method of the present invention can observe follicular or parafollicular cells or both follicular and parafollicular cells.

Thyroid biological samples can be thyroid cells or tissues obtained for example by fine needle aspiration or by surgery.

According to the method of the invention the step of detecting the localization of FBXO34 protein can be carried out by an immunostaining technique such as for example immunohistochemistry, immunocytochemistry, immunofluorescence, western blotting, flow cytometry, enzyme-linked immunosorbent assay or electron microscopy.

The present invention now will be described by an illustrative, but not limitative way, according to preferred embodiments thereof, with particular reference to enclosed drawings, wherein:

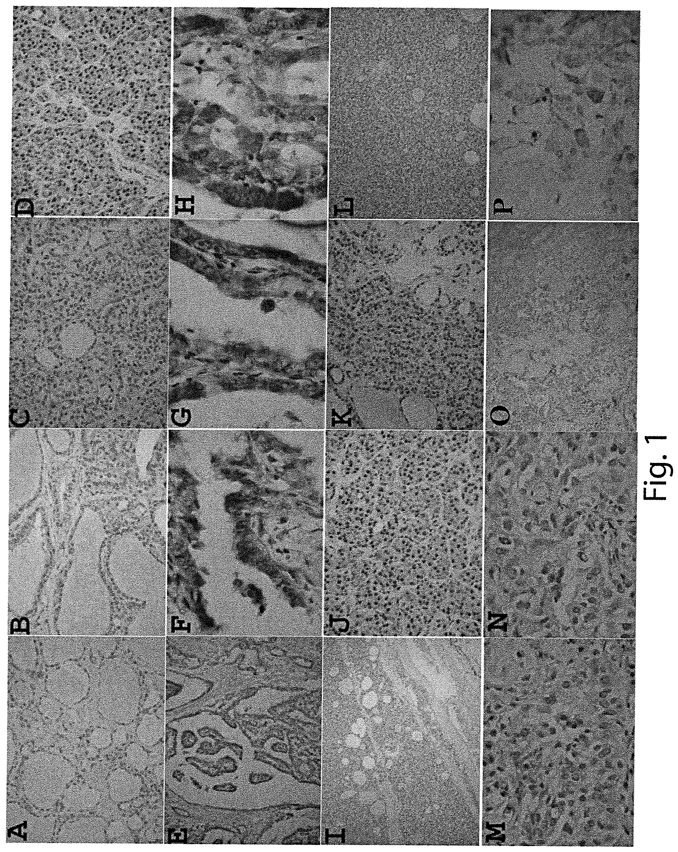

**Figure 1** shows A: nuclear FBXO34 immunoreactions of in normal thyroid tissue. B: nuclear immunolocalization of FBXO34 in goiter. C and D: immunoreactions of FBXO34 in the nuclei of FA and OA cells respectively. E-H: immunoreactions of FBXO34 in nucleus and cytoplasm of PTC cells. I-L: nuclear and cytoplasmatic FBXO34 immunoreactions in minimally invasive FTC. M-N: nuclear and cytoplasmatic FBXO34 immunoreactions in an ATC O-P nuclear and cytoplasmatic FBXO34 immunoreactions in an MTC.

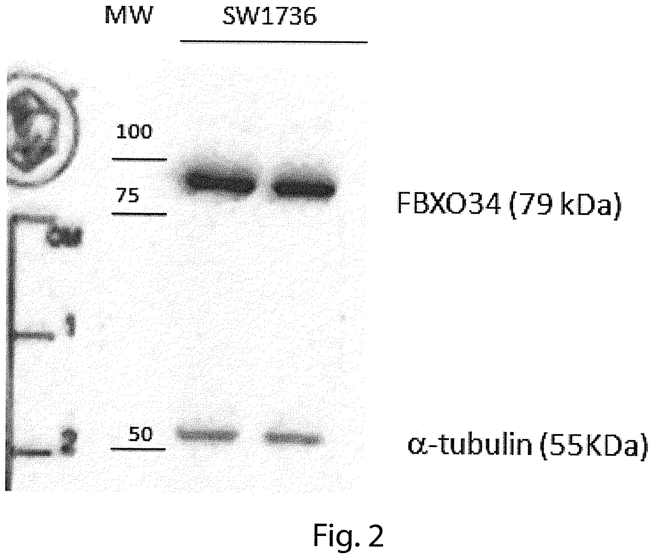

**Figure 2** shows western blot analysis of total extract (without distinction between nucleus and cytoplasm) from SW1736 cell line, derived from a human anaplastic thyroid carcinoma. The figure shows the band of the expected size of the FBX034 protein (79KDa), characterized by high intensity, compared to the α-tubulin, used as a control.

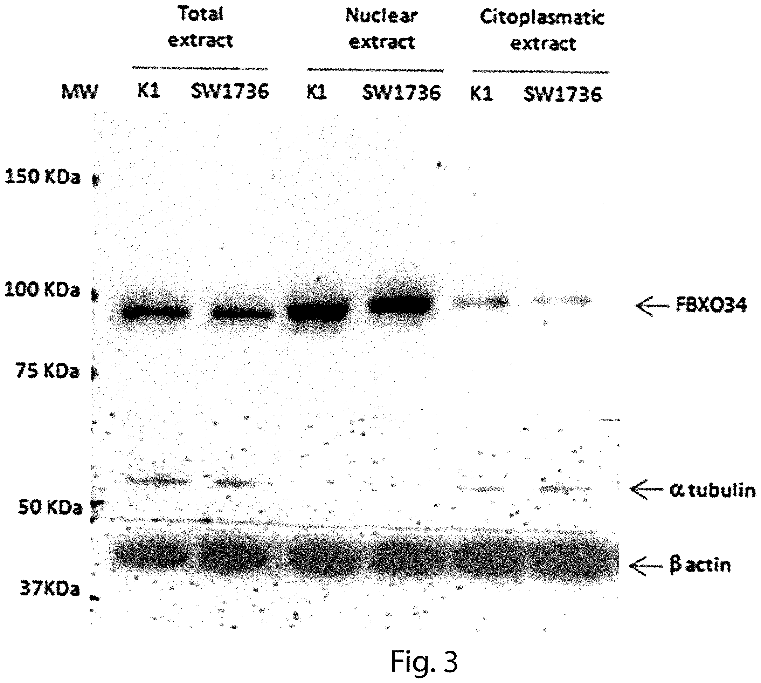

**Figure 3** shows western blot analysis of total cell extracts from the two thyroid cell lines, namely the K1, derived from thyroid papillary carcinoma and the SW1736, derived from anaplastic thyroid carcinoma, after subcellular fractioning. FBXO34 protein is detected in the nuclear extract (higher expression level) and in the cytoplasmic extract (lower expression level). The ubiquitously expressed β-actin and the nucleus-specific expressed α-tubulin proteins were used as controls.

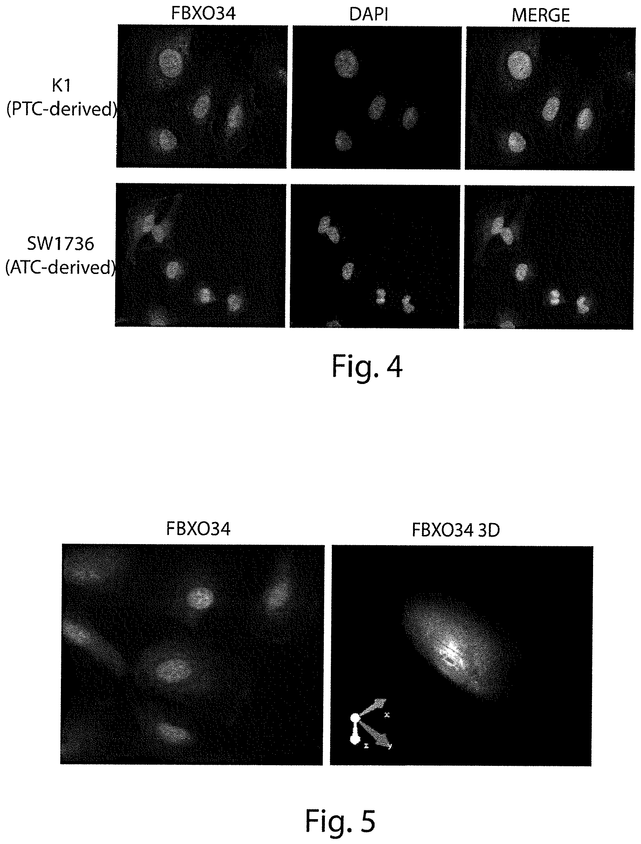

**Figure 4** shows immunofluorescence analysis of K1 and SW1736 cells. The FBX034 expression is visible as red signal, while the blue dye Hoechst was used to counterstain the DNA inside the nucleus. A high and diffuse expression of the FBXO34 protein throughout the entire cytoplasm and a more particulate expression inside the nucleus are showed.
**Figure 5** shows nuclear as well the cytoplasmic localization in K1 cells by the 3D image of the immunofluorescence.

### EXAMPLE 1: Analysis of the expression of FBXO34 protein in human thyroid tissues

### Patients and Tissues Collection

A total of 72 thyroid glands, surgically removed and collected at the Unit of Integrated Ultrastructural Pathology, Department of Human Pathology, University of Messina was examined. The surgical samples were obtained from patients evaluated and followed up at the Endocrine Unit of the University of Messina. All patients (52 females and 20 males, aged 28 to 82 years, mean 54.1 ± 15.1 years) were euthyroid at the time of the thyroidectomy. Thirtytwo of 72 examined glands showed two lesions enclosing a goitrous nodule, associated with a benign or malignant lesion. In the remaining 40 thyroid glands a solitary nodule was observed. A total number of 104 lesions was analyzed, consisting of 37 goiters (TG), 30 adenomas, of which 19 was follicular (FA) and 11 oncocytic types (OA) and 29 37 carcinomas including 14 papillary (PTC), 10 follicular (FTC, 5 anaplastic (ATC) and 8 medullary (MTC) variants. Informed consent was obtained and the study was approved by the local Ethics Committee.

The pathological features of lesions are listed in **Table 1.** According to standard criteria of the Armed Forces Institute of Pathology (17), the 37 goiters were classified in colloid (n= 19) and parenchymatous (n= 18) while the 19 FA included four variants such as normofollicular (n= 5), macrofollicular (n= 5), microfollicular (n= 6) and solid (n= 3). Each lesion sample was treated separately for immunohistochemistry analysis.

Thyroid tissues were fixed in 4% neutral buffered formalin and embedded in paraffin. Serial sections of each lesion were stained with hematoxylin and eosin (H&E) and Periodic acid Schiff (PAS) histochemical staining and the slides were reviewed to confirm the diagnosis. The immunoreactivities for FBXO34 were graded as I, II, III, IV, V and VI by using "Quickscore" method as proposed from Detre et al. (18) that scores the stained cells in a system from I to VI (I = 0-4% of cells; II = 5-19%; III = 20-39%; IV = 40-59%; V = 60-79%; VI = 80-100%).

### Reagents and Immunohistochemical Procedure

Immunohistochemical staining was carried out on sections of 5-µm thick of the selected blocks. Immunohistochemistry was performed using a polyclonal rabbit antibody against a fragment of FBXO34 - C-terminal located between amino acids 607-637 and recognizing the SCF (SKP1-CUL1-F-box protein)-type E3 ubiquitin ligase complex. (ab175296, 1:100; Abcam Cambridge, UK; http://www.abcam.com/fbxo34-antibody-c-terminal-ab175296.html)
Antigen retrieval technique was carried out as described (19). Tissue sections were deparaffinized in xylene and rehydrated in alcohol. Then, the endogenous biotin was inactivated by addition of 0.05% (v/v) solution of streptavidin in phosphate-buffered saline (PBS) and the endogenous peroxidase activity was quenched by adding 0.3% (v/v) solution of 3% H₂O₂/methanol for 30 min. Immunostaining was obtained with the LSAB system (Universal Dako LSAB® + Kit, Peroxidase, Carpinteria, CA, USA) by using an automated slide processing system designed from Autostainer Link 48 system (Dako). The reaction was visualized by using 3,3'-diaminobenzidine (Dako) activated with 0.05% hydrogen peroxide. Sections were counterstained with Mayer's haematoxylin, dehydrated and mounted. Specificity of the binding was assessed on appropriate negative controls, either by omitting the primary antiserum or by replacing the primary antiserum with normal rabbit serum. In addition, an immunoabsorption test was performed to confirm the specific immunoreactivity of the antibody. Specimens of colon carcinoma were used as appropriate positive controls for FBXO34 antibody. Subcellular location of FBXO34 immunereactivity was assessed sharing cytoplasm and nucleus.

### Immunohistochemical Results

The results obtained with immunohistochemical analysis of thyroid lesions are resumed in **Table 1** and **Table 2** and representative picture of the immunohistochemistry results for each type of thyroid tumor as well as for normal thyroid tissue are shown in **Figure 1****.**

**Table 1**

| | **Cellular Immunodistribution of FBXO34 protein** | | | | | |
|---|---|---|---|---|---|---|
| | I % of cases | II % of cases | III % of cases | IV % of cases | V % of cases | VI % of cases |
| Normal n. 40 | 20 | 27.5 | 47.5 | 2.5 | 2.5 | 0 |
| Goiter n. 37 | 0 | 5 | 49 | 30 | 16 | 0 |
| FA n. 19 | 0 | 0 | 0 | 6 | 47 | 47 |
| OA n.11 | 0 | 0 | 0 | 27 | 46 | 27 |
| PTC n. 14 | 0 | 0 | 0 | 0 | 14 | 86 |
| FTC n. 10 | 0 | 0 | 0 | 0 | 40 | 60 |
| ATC n. 5 | 0 | 0 | 0 | 0 | 0 | 100 |
| MTC n.8 | 12.5 | | 0 | 0 | 62.5 | 25 |

**Table 2**

| | **Subcellular Immunodistribution of FBXO34 protein** | |
|---|---|---|
| | Nucleus % of cases | Cytoplasm % of cases |
| Normal | 100 | 0 |
| Goiter | 100 | 0 |
| FA | 100 | 0 |
| OA | 100 | 0 |
| PTC | 100 | 100 |
| FTC | 100 | 100 |
| ATC | 100 | 100 |
| MTC | 100 | 100 |

FBXO34 was expressed either in normal human thyroid tissues or benign and malignant lesions. However, in malignant lesions such as PTC, FTC, ATC and MTC, the immunoreaction of FBXO34 was located in both nucleus and cytoplasm. In sharp contrast, FBXO34 immunostaining was absent or appeared solely in the nuclear compartment of normal adjacent thyroid tissues as well as in benign nodules of goiters, in FAs and in OAs. The diagnostic performance of the specific subcellular localization of FBXO34 in the cytoplasm is reported in **Table 3** which shows the diagnostic performance of the specific FBXO34 cytoplasmic localization by IHC analysis.

**Table 3**

| | Sensitivity | Specificity | PPV | NPV | Accuracy |
|---|---|---|---|---|---|
| FBXO34 by IHC | 100% | 100% | 100% | 100% | 100% |

### EXAMPLE 2: Analysis of FBXO34 protein expression in human thyroid carcinoma cell lines

### Cell culture

The FBXO34 protein levels were analyzed in two different human thyroid tumor cell lines, namely the K1 (ECACC, Sigma Aldrich, #92030501) derived from a human papillary thyroid carcinoma and the SW1736 (CLS, Cell Lines Service, GmBH, #300453), derived from a human anaplastic thyroid carcinoma. Cells were grown on RPMI 1640 culture media (Euroclone, #ECM2001-L), supplemented with 10% Foetal Bovine Serum (FBS) (Euroclone, #ECM0170-L), 2 mM glutamine, and with 100 U penicillin / 0.1 mg/ml streptomycin (Euroclone, #ECB3001-D).

### Reagents and Western Blot Procedure

Total cell proteins were extracted from 10⁶ cells using RIPA buffer (Sigma-Aldrich) with 1X Complete Protease Inhibitor Cocktail (Roche). A total of 40 µg of proteins were separated by 8% SDS-PAGE and transferred to nitrocellulose membranes (Amersham, GE Healthcare). Membrane with proteins was blocked by 5% non-fat milk in T-PBS buffer (PBS 1x, 0,05% Tween 20) and then incubated with primary anti-FBXO34 antibody (orb35983, rabbit, polyclonal, dilution 1:1000, Biorbyt) overnight at 4°C. After the incubation, the membrane was washed three times with T-PBS buffer for 5 minutes each and then incubated with goat anti-rabbit IgG-HRP secondary antibody (sc-2004, dilution 1:4000, Santa Cruz). After three washing with T-PBS buffer, the signal was detected by chemiluminescence with ECL (Roche) and visualized using ChemiDoc-it (UVP). The anti-aTubulin primary antibody (sc-8035, mouse monoclonal, dilution 1:1000, Santa Cruz) and the goat anti-mouse IgG-HRP secondary antibody were used for the normalization of the FBXO34 signals.

### Analysis of the Subcellular localization

The separation of nuclear protein extract from the cytoplasmic fraction from cell lines was performed using the Nuclear/Cytosol Fractionation Kit (Catalog No. JM-K266, MBL), according to the manufacturer's guidelines. A total of 10⁶ cells were collected by centrifugation at 1200 x g for 5 minutes at 4°C and the cell pellets were resuspend in 0.2 ml of CEB-A Mix containing DTT and Protease Inhibitors, supplied in the kit. After the addition of 11 µl of ice-cold Cytosol Extraction Buffer-B, the samples were centrifuged for 5 minutes at 4°C at maximal speed (16,000 x g) and the supernatant fraction (cytoplasmic extract) were immediately transfered to a clean pre-chilled tube. The pellets were resuspended in 100 µl of ice-cold Nuclear Extraction Buffer Mix (NEB mix) and vortexed on the highest setting for 15 seconds every 10 minutes, for a total 40 minutes. After a centrifugation at full speed (16,000 x g) for 10 minutes at 4°C, the supernatant (nuclear extract) were transferred to a clean pre-chilled tube. The protein content in each fraction was determined by Bio-Rad Protein Assay Dye Reagent Concentrate (cod. 500-006 BIO-RAD).

### Western blot Results

Western blot analysis, performed using total extract from SW1736 thyroid carcinoma cell line, showed a unique band of the expected size at 79 KDa (Fig. 2). The band was intense, compared to the α-tubulin, used to normalize the expression level of the protein.

Western blot analysis was performed also using total cell extracts from the two thyroid cell lines K1 and SW1736, after subcellular fractioning. In both human-derived thyroid carcinoma cell lines the FBXO34 protein is localized in the nucleus (higher expression level) and in the cytoplasm (lower expression level). The ubiquitously expressed β-actin and the nucleus-specific expressed α-tubulin proteins were used as controls (**Fig**. **3**).

### EXAMPLE 3: Immunofluorescent analysis

### Reagents and Immunofluorescent Procedure

The two human-derived thyroid cell lines, K1 and SW1736 were utilized also for immunofluorescence analysis. Approximately 150,000 cells were seeded on coverslip slides, pretreated with Poly-L-lysine 0.01% solution (Sigma-Aldrich), mounted onto a 35 mm-dish and let grow for 24 hours in RPMI-1640, added with 10% FBS, 100 U/ml of penicillin, and 100 µg/ml of streptomycin.

Cells were then washed with PBS to remove tissue culture medium and fixed for 5 min. at -20°C with absolute methanol. After a brief washing with PBS cells were permeabilized for 10 min. at room temperature with 0.25% Triton-X100 in PBS. After another washing step in PBS, cells were incubated for 60 min. at room temperature with bovine serum albumin (BSA) 5% in PBS to saturate aspecific bindings of the antibody. Cells were then incubated overnight at 4°C in humid chamber with the specific primary antibody anti-FBXO34 (Abcam ab#175296) rabbit, polyclonal, diluted 1/30 in 5% BSA in PBS. After 3 washing steps in PBS, cells were incubated for 1 hour at 37°C, in the dark with the secondary antibody, conjugated with a fluorophore, donkey anti-rabbit Alexa Fluor 568 (Termo Fisher Scientific #A10042), at the concentration of 5µg/ml, diluted in 5% BSA in PBS. Slides were, then, washed 3 times in PBS. Cell nuclei were counterstained with the DNA specific dye Hoechst (Bisbenzimide H33258 Sigma-Aldrich) for 5 min. in the dark at room temperature. After washing with PBS the slides were mounted on a glass slide using the mounting medium Mowiol 4-88 (Sigma-Aldrich) and stored in the dark at 4°C.

Analysis of the slides was performed using the inverted microscope Nikon Eclipse Ti-E, equipped with the fluorescent light Intensilight and with appropriate filters that transmit the desired wavelengths. Images were captured using the camera Andor Neo, with an oil immersion lens 60x. Three-dimensional images were obtained using the Z-stack function and processed with the computer software Nikon Nis_Elements C.

### Immunofluorescent Results

The two human-derived thyroid carcinoma cell lines showed a high and diffuse expression of the FBXO34 protein throughout the entire cytoplasm and a more particulate expression inside the nucleus (Fig. 4). The nuclear as well the cytoplasmic localization were confirmed in K1 cells by the 3D image of the immunofluorescence (**Fig**. **5**).

### EXAMPLE 4: Application of the method of the present invention to thyroid FNA-cytology

The immuno-detection of FBXO34 protein expression in the cytoplasm can be performed on thyroid fine needle aspiration (FNA) cytological specimens alone or in combination with other known thyroid cancer markers, such as Galectin-3 and HBME-1, to increase the accuracy of conventional FNA cytology.

### Collection of thyroid FNA specimens

FNA-cytology, performed under US guidance, represents the most important pre-surgical diagnostic method to diagnose the nature of a thyroid nodule. Thyroid FNA samples, obtained from each thyroid nodule, are directly rinsed by the needle into a 15 ml Falcon tube, containing buffered-formalin and cell-block preparation is performed as previously described (20). Thyroid cells obtained by FNA are collected into a 15 mL Falcon tube filled with 8-10 mL of physiological saline solution or PBS, centrifuged gently at 700-1000 RPM for 10 min and the supernatant is decanted off. The pellet is re-suspend in 3-4 drops of plasma. Few drops (3-4 drops) of thromboplastin and few drops (3-4 drops) of calcium chloride are added and then mixed gently for a faster fibrin clot reaction. Then 5-10 mL of buffered formalin are added and the samples are incubated for 12-24 hrs to allow them to fix. After spinning down gently the clot are placed in a labelled tissue cassette and processed like a small tissue biopsy for cellblock preparation. Slides are stained in haematoxilin/eosin for the routine histo-cytological evaluation.

### Immunocytochemical analysis of FBXO34 on thyroid FNA specimens

FBKO34 immunostaining are performed on 4 µm consecutive sections, using polyclonal anti human FBXO34 antibody (orb35983, rabbit, polyclonal, Biorbyt, Cambridge, UK) at dilution of 1:1000. Antigen retrieval was obtained by microwave treatment at 750W in 0.01M citrate buffer, at pH 6 for 3 cycles of 3 to 5 min each. FBXO34 immunostaining is considered positive when follicular thyroid cells show FBXO34 accumulation in the cytoplasm, with or without nuclear staining. Nodules that show no evidence of cytoplasmic staining for FBXO34 are considered negatives for malignancy. Nuclear expression of FBXO34 is considered as internal positive controls. An original algorithm is developed to analyze the immunocytochemical data and to give the final results of the test.

### REFERENCES

**1.** Hegedüs L. Clinical practice. The thyroid nodule. N. Engl. J. Med. 351(17), 1764-1771, 2004.
**2.** Davies L, Welch H.G. Increasing incidence of thyroid cancer in the United States, 1973-2002. JAMA. 10;295(18), 2164-267, 2006.
**3.** Marqusee E, Benson CB, Frates MC, Doubilet PM, Larsen PR, Cibas ES, Mandel SJ. Usefulness of ultrasonography in the management of nodular thyroid disease. Ann Intern Med. 7;133(9):696-700, 2000.
**4.** Pellegriti G, Frasca F, Regalbuto C, Squatrito S, Vigneri R. Worldwide increasing incidence of thyroid cancer: update on epidemiology and risk factors. Cancer Epidemiol.2013:965212. doi: 10.1155/2013/965212, 2013.
**5.** Dal Maso L, Lise M, Zambon P, Falcini F, Crocetti E, Serraino D, Cirilli C, Zanetti R, Vercelli M, Ferretti S, Stracci F, De Lisi V, Busco S, Tagliabue G, Budroni M, Tumino R, Giacomin A, Franceschi S; AIRTUM Working Group. Incidence of thyroid cancer in Italy, 1991-2005: time trends and age-period-cohort effects. Ann Oncol, 22(4):957-63. doi: 10.1093/annonc/mdq467, 2011.
**6.** Sakorafas GH, Peros G, Farley DR. Thyroid nodules: does the suspicion for malignancy really justify the increased thyroidectomy rates? Surg Oncol, 15(1):43-55, 2006.
**7.** Bilimoria KY, Zanocco K, Sturgeon C. Impact of surgical treatment on outcomes for papillary thyroid cancer. Adv Surg, 42:1-12, 2008.
**8.** Kocjan G, Feichter G, Hagmar B, Kapila K, Kardum-Skelin I, Kloboves V, Kobayashi TK, Koutselini H, Majak B, Schenck U, Schmitt F, Tani E, Totch M, Onal B, Vass L, Vielh P, Weynand B, Herbert A. Fine needle aspiration cytology: a survey of current European practice. Cytopathology. 17(5):219-26, 2006.
**9.** Haugen Bryan R., Alexander Erik K., Bible Keith C., Doherty Gerard M., Mandel Susan J., Nikiforov Yuri E., Pacini Furio, Randolph Gregory W., Sawka Anna M., Schlumberger Martin, Schuff Kathryn G., Sherman Steven I., Sosa Julie Ann, Steward David L., Tuttle R. Michael, and Wartofsky Leonard.2015 American Thyroid Association Management Guidelines for Adult Patients with Thyroid Nodules and Differentiated Thyroid Cancer. American Thyroid Association (ATA) Guidelines Taskforce on Thyroid Nodules and Differentiated Thyroid Cancer. Thyroid. Jan 2016, 26(1): 1-133.
**10.** Cibas ES, Ali SZ. The Bethesda System for Reporting Thyroid Cytopathology. Thyroid. 19(11):1159-165. doi: 10.1089/thy.2009.0274, 2009.
**11.** Cochand-Priollet B, Schmitt FC, Tötsch M, Vielh P. European Federation of Cytology Societies' Scientific Committee. The Bethesda terminology for reporting thyroid cytopathology: from theory to practice in Europe. Acta Cytol. 55(6):507-11, 2011.
**12.** Chapter IV, Trovato MC, "Cancer Thyroid Nodule: Knowing is Doing", in the book entitled "Thyroid Nodules: Risk Factors, Diagnosis and Management", Editor Marsha Vasquez; Series: Endocrinology Research and Clinical Developments (ISBN: ebook 978-1-63483-615-9; H-cover 978-1-63483-592-3), 2015 Ed. Nova Science Publishers, Inc. Hauppauge New York, USA.
**13.** Jianping Jin, Timothy Cardozo, Ruth C. Lovering, Stephen J. Elledge, Michele Pagano, and J. Wade Harper. Systematic analysis and nomenclature of mammalian F-box proteins. Genes Dev. 2004 Nov 1; 18(21): 2573-2580.
**14.** Bai C, Sen P, Hofmann K, Ma L, Goebl M, Harper JW, Elledge SJ SKP1 connects cell cycle regulators to the ubiquitin proteolysis machinery through a novel motif, the F-box. Cell. 1996 Jul 26; 86(2):263-74.
**15.** Skaar JR, Pagan JK, Pagano M (2013) Mechanisms and function of substrate recruitment by F-box proteins. Nat Rev Mol Cell Biol 14(6):369-381.
**16.** Kipreos ET1, Pagano M. The F-box protein family. Genome Biol. 2000; 1(5): reviews3002.1-reviews3002.7. Epub 2000 Nov 10.
**17.** Juan Rosai, Ronald A. Delellis, Maria Luisa Carcangiu, William J. Frable, Giovanni Tallini. Tumors of the Thyroid and Parathyroid Glands (AFIP Atlas of Tumor Pathology Series 4) Hardcover - March 27, 2015.
**18.** S Detre, G Saclani Jotti, and M Dowsett J Clin Pathol. 1995 Sep; 48(9): 876-878. A "quickscore" method for immunohistochemical semiquantitation: validation for oestrogen receptor in breast carcinomas.
**19.** Shan-Rong Shi,Yan Shi, and Clive R.Taylor. Antigen Retrieval Immunohistochemistry: Review and Future Prospects in Research and Diagnosis over Two Decades. Journal of Histochemistry & Cytochemistry 59(1) 13-32, 2011.
**20.** Bartolazzi A, Bellotti C, Sciacchitano S. Methodology and technical requirements of the galectin-3 test for the preoperative characterization of thyroid nodules. Appl Immunohistochem Mol Morphol. 2012 Jan;20(1):2-7.

## Claims

1. *In vitro* use of FBXO34 protein as a marker of thyroid carcinoma, wherein said protein is localized in both cytoplasm and nucleus of thyroid cells in thyroid carcinoma.

2. Use according to claim 1, wherein said thyroid carcinoma is chosen from the group consisting of papillary, follicular, anaplastic or medullary thyroid carcinoma.

3. Use according to anyone of claims 1-2, wherein the thyroid cells are chosen from the group consisting of follicular and/or parafollicular cells.

4. Method for *in vitro* diagnosis of thyroid carcinoma, said method comprising or consisting of detecting the localization of FBXO34 protein in cytoplasm, and optionally in nucleus, of thyroid cells in a thyroid biological sample, wherein said protein is localized in both the cytoplasm and the nucleus of thyroid carcinoma cells.

5. Method according to claim 4, wherein said thyroid carcinoma is chosen from the group consisting of papillary, follicular, anaplastic or medullary carcinoma.

6. Method according to anyone of claims 4-5, wherein the thyroid cells are chosen from the group consisting of follicular and/or parafollicular cells.

7. Method according to anyone of claims 4-6, wherein said step of detecting the localization of FBXO34 protein is carried out by an immunostaining technique.

8. Method according to claim 7, wherein said an immunostaining technique is chosen from the group consisting of immunohistochemistry, immunocytochemistry, immunofluorescence, western blotting, flow cytometry, enzyme-linked immunosorbent assay, electron microscopy.

9. Method according to anyone of claims 4-8, wherein the thyroid biological sample is chosen from the group consisting of thyroid cells or thyroid tissue.

## Patentansprüche

1. *In-vitro*-Verwendung eines FBXO34-Proteins als Marker eines Schilddrüsenkarzinoms, wobei das Protein in beiden, Zytoplasma und Kern von Schilddrüsenzellen bei einem Schilddrüsenkarzinom lokalisiert wird.

2. Verwendung nach Anspruch 1, wobei das Schilddrüsenkarzinom aus der Gruppe ausgewählt wird, die aus papillärem, follikluärem, anaplastischem oder medullärm Schilddrüsenkarzinom besteht.

3. Verwendung nach einem der Ansprüche 1-2, wobei die Schilddrüsenzellen aus der Gruppe ausgewählt werden, die aus follikluären und/ oder parafollikluären Zellen besteht.

4. Verfahren zur *In-vitro*-Diagnose eines Schilddrüsenkarzinoms, wobei das Verfahren das Detektieren der Lokalisierung eines FBXO34-Proteins im Zytoplasma, und gegebenenfalls im Kern von Schilddrüsenzellen in einer biologischen Schilddrüsenprobe umfasst oder daraus besteht, wobei das Protein in beiden, dem Zytoplasma und dem Kern von Schilddrüsenkarzinomzellen lokalisiert wird.

5. Verfahren nach Anspruch 4, wobei das das Schilddrüsenkarzinom aus der Gruppe ausgewählt wird, die aus papillärem, follikluärem, anaplastischem oder medullärm Karzinom besteht.

6. Verfahren nach einem der Ansprüche 4-5, wobei die Schilddrüsenzellen aus der Gruppe ausgewählt werden, die aus follikluären und/ oder parafollikluären Zellen besteht.

7. Verfahren nach einem der Ansprüche 4-6, wobei der Schritt des Detektierens der Lokalisierung eines FBXO34-Proteins durch eine Immunfärbungstechnik ausgeführt wird.

8. Verfahren nach Anspruch 7, wobei die Immunfärbungstechnik aus der Gruppe ausgewählt wird, die aus Immunhistochemie, Immunzytochemie, Immunfluoreszenz, Western Blotting, Durchflusszytometrie, enzymverbundene Immunanalyse, Elektronenmikroskopie besteht.

9. Verfahren nach einem der Ansprüche 4-8, wobei die biologische Schilddrüsenprobe aus der Gruppe ausgewählt wird, die aus Schilddrüsenzellen oder Schilddrüsengewebe besteht.

## Revendications

1. Utilisation in vitro de protéine FBXO34 en tant qu'un marqueur de carcinome thyroïdien, dans laquelle ladite protéine est localisée à la fois dans le cytoplasme et dans le noyau de cellules thyroïdiennes dans un carcinome thyroïdien.

2. Utilisation selon la revendication 1, dans laquelle ledit carcinome thyroïdien est choisi dans le groupe se composant de carcinomes thyroïdiens papillaires, folliculaires, anaplasiques ou médullaires.

3. Utilisation selon la revendication 1-2, dans laquelle les cellules thyroïdiennes sont choisies dans le groupe se composant de cellules folliculaires et/ou parafolliculaires.

4. Procédé de diagnostic *in vitro* de carcinome thyroïdien, ledit procédé comprenant ou se composant de la détection de la localisation de protéine FBXO34 dans le cytoplasme, et facultativement dans le noyau, de cellules thyroïdiennes dans un échantillon biologique thyroïdien, dans lequel ladite protéine est localisée à la fois dans le cytoplasme et dans le noyau de cellules de carcinome thyroïdien.

5. Procédé selon la revendication 4, dans lequel ledit carcinome thyroïdien est choisi dans le groupe se composant de carcinomes thyroïdiens papillaires, folliculaires, anaplasiques ou médullaires.

6. Procédé selon la revendication 4-5, dans lequel les cellules thyroïdiennes sont choisies dans le groupe se composant de cellules folliculaires et/ou parafolliculaires.

7. Procédé selon l'une quelconque des revendications 4-6, dans lequel ladite étape de la détection de la localisation de protéine FBXO34 est effectuée par une technique d'immunocoloration.

8. Procédé selon la revendication 7, dans lequel ladite technique d'immunocoloration est choisie dans le groupe se composant d'immunohistochimie, d'immunocytochimie, d'immunofluorescence, de transfert de Western, de cytométrie en flux, d'essai d'immuno-absorption enzymatique, et de microscopie électronique.

9. Procédé selon l'une quelconque des revendications 4-8, dans lequel l'échantillon biologique thyroïdien est choisi dans le groupe se composant de cellules thyroïdiennes ou de tissus thyroïdiens.
